Europäisches Patentamt

European Patent Office

Office européen des brevets

(19)

(11) Veröffentlichungsnummer: **0 266 688**
**A2**

(12) **EUROPÄISCHE PATENTANMELDUNG**

(21) Anmeldenummer: 87115953.9

(22) Anmeldetag: 30.10.87

(51) Int. Cl.⁴: **A61L 2/26**

(30) Priorität: 05.11.86 DE 3637687

(43) Veröffentlichungstag der Anmeldung:
**11.05.88 Patentblatt 88/19**

(84) Benannte Vertragsstaaten:
**AT BE CH DE FR GB IT LI NL SE**

(71) Anmelder: **BASF Aktiengesellschaft**
**Carl-Bosch-Strasse 38**
**D-6700 Ludwigshafen(DE)**

(72) Erfinder: **Blinne, Gerd, Dr.**
**Im Woogtal 7**
**D-6719 Bobenheim(DE)**
Erfinder: **Buchert, Hermann, Dr.**
**An der Nolzeruhe 8 c**
**D-6702 Bad Duerkheim(DE)**
Erfinder: **Kroebl, Karl, Dr.**
**Kaiserslauterer Strasse 28**
**D-6702 Bad Duerkheim(DE)**
Erfinder: **Welz, Martin**
**Kaiserslauterer Strasse 231**
**D-6702 Bad Duerkheim(DE)**
Erfinder: **Zeiner, Hartmut, Dr.**
**Johann-Strauss-Strasse 13**
**D-6831 Plankstadt(DE)**

(54) **Behälter für medizinisches Material.**

(57) Der Behälter (1) ist an seiner Öffnung (6) mit
einer hermetisch abschließenden und wieder abziehbaren Abdeckung (5) aus Folienmaterial, das auch
faserverstärkt sein kann, versehen, das undurchlässig ist für biologische Keime und
durchlässig ist für sterilisierende Mittel.

**EP 0 266 688 A2**

## Behälter für medizinisches Material

Die Erfindung betrifft einen Behälter zum Aufbewahren und Sterilisieren von medizinischem Material.

Behälter für z.B. Prothesen, Verbandsmaterial, chirurgische Geräte usw. sollten nach Einlegen der Materialien so dicht verschlossen werden, daß zwar keine Keime eindringen können, aber eine Sterilisation durch z.B. eindringenden Wasserdampf, Ethylenoxid und/oder γ-Strahlen möglich ist. Dazu wurde schon versucht, eine entsprechende poröse Membran, beispielsweise aus Papier mit abgestimmter Porengröße auf thermoplastische Behälter aufzukleben. Die Klebschichten sind jedoch nicht gegen Wasserdampf beständig und die Ausführung bei der geforderten Sorgfalt aufwendig. Wesentlich einfacher ist das Aufschweißen des Papiers auf den Behälter. Durch eine Beschichtung des Papiers im Schweißnahtbereich mit einem mit dem Thermoplast verschweißbaren Material konnte dabei auch eine gute Haftfestigkeit erzielt werden.

Beim Abtrennen des Papiers zur Entnahme des Inhalts reißt jedoch das Papier auf, wobei Flusen freigesetzt werden, die in einem sterilen Operationsraum schädlich sein können. Werden aber die Schweißbedingungen so gewählt, daß ein flusenfreies Abziehen des Papiers möglich ist, ergibt sich eine so schwache Verbindung, daß ein unbeabsichtigtes Öffnen des Behälters möglich ist.

Es bestand daher die Aufgabe, einen Behälter zu entwickeln, bei dem eine poröse Membranabdeckung ohne die vorstehend geschilderten Nachteile einsetzbar ist.

Die Lösung der Aufgabe besteht darin, daß eine die Öffnung des Behälters hermetisch abschließende Abdeckung aus folienartigen Material vorgesehen ist, welche ein Fenster mit einer porösen Membran aufweist, das undurchlässig ist für biologische Keime und durchlässig ist für sterilisierende Mittel.

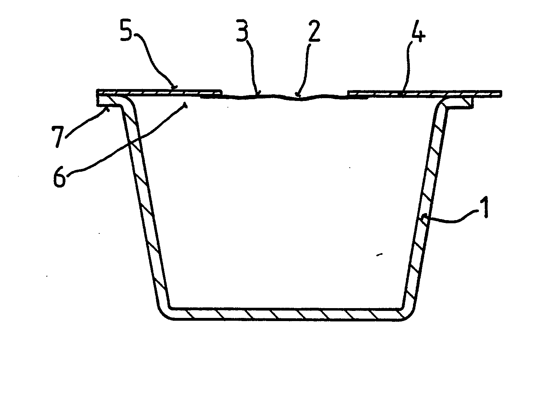

Ein Ausführungsbeispiel des erfindungsgemäßen Behälters ist anhand der Zeichnung nachfolgend beschrieben.

In eine Folie 4 mit der äußeren Kontur des Behälters 1 werden eine oder mehrere Öffnungen 2 gestanzt, deren Fläche dem notwendigen Einlaßquerschnitt für das sterilisierende Medium (z.B. Wasserdampf) entspricht. Über diese Öffnungen werden entsprechend größere poröse Membranen, beispielsweise Zuschnitte aus Papier 3 gelegt und fest mit der Folie 4 verschweißt. Dabei kann zusätzlich durch einen Folienring das Papier am Rand abgedeckt werden. Die so präparierte Folienabdeckung 5 mit dem Papierfenster 3 wird dann mit dem die Behälteröffnung 6 umlaufenden Flansch 7 des Behälters so verschweißt, daß die Folie von Hand abgezogen werden kann.

Als für biologische Keime undurchlässiges und für sterilisierende Mittel durchlässiges Fenster wird ein für diese Zwecke geeignetes handelsübliches Papier eingesetzt, das die Anforderungen hinsichtlich Sterilisationsbedingungen und Porengröße erfüllt. Zur Verbesserung der Schweißbarkeit kann es im Bereich der Schweißnaht mit einer Lösung eines Thermoplasten getränkt sein, der mit dem Folienmaterial gut verschweißbar ist. Der Thermoplastauftrag beträgt dabei 10 bis 60 g/m².

Weiterhin sind Filtermembranen aus beispielsweise Polysulfon geeignet. Sie sind sehr gut mit Polysulfonfolien verschweißbar und benötigen keine Vorbehandlung.

Als Material sowohl für den spritzgegossenen oder aus Halbzeug warmgeformten Behälter wie auch für die Folie können folgende Thermoplaste eingesetzt werden: Polyamide, Polysulfon, Polyethersulfon, vorzugsweise Polysulfon.

Die Folie hat eine Dicke zwischen 10 und 2000 μm, vorzugsweise 50 bis 300 μm; sie kann durch Extrusion, Blasen, Gießen oder ein anderes übliches Verfahren hergestellt sein und sollte möglichst wenig Eigenspannungen aufweisen, um Probleme beim Schweißen und Sterilisieren zu vermeiden.

In einer weiteren, vorteilhaften Ausführungsform des erfindungsgemäßen Behälters ist das Folienmaterial zur Erhöhung der Reißfestigkeit faserverstärkt, dessen Dicke dann zwischen 50 und 1000 μm betragen kann. Die Fasern können aus synthetischen oder natürlichen Stoffen wie Glas, Baumwolle, aromatischen Polyamiden, Kohlenstoff o.ä. bestehen und sollten mindestens 5 mm lang sein. Sie können in Form eines Vlieses bzw. einer Matte oder eines Gewebes eingesetzt werden. Das Flächengewicht dieser textilen Gebilde liegt zwischen 20 und 300 g/m², vorzugsweise zwischen 30 und 100 g/m². Der Faseranteil des verstärkten thermoplastischen Materials liegt zwischen 10 und 80 Gew.%, vorzugsweise zwischen 40 und 70 %. Die Tränkung des textilen Gebildes mit der thermoplastischen Matrix erfolgt z.B. durch Tränken mit einer Lösung des Thermoplasten und anschließendes Verdampfen des Lösungsmittels oder durch Beschichten mit einer Thermoplastschmelze, die zwischen beheizten Walzen in das Textilgebilde eingepreßt wird. Es sollte mindestens soviel thermoplastisches Material aufgetragen werden, daß eine geschlossene, porenfreie Schicht entsteht.

Das Papier bzw. Membranmaterial hat ein Flächengewicht im Bereich von 30 bis 200 g/m², vorzugsweise 50 bis 100 g/m².

Zum Verschweißen des Abdeckungs-und Papier-bzw. Membranzuschnittes können verschiedene Schweißverfahren eingesetzt werden, beispielsweise Ultraschallschweißverfahren, Wärmekontaktverfahren, Wärmeimpulsverfahren.

Für das Ultraschallschweißen sind Amplituden von 30 bis 55 μm, Schweißzeiten von 0,2 bis 2 sec und Drücke von 1 bis 5 bar zweckmäßig. Die optimalen Bedingungen sind auf die Materialien abzustimmen. Dabei können Schälfestigkeiten von über 2 N pro mm Schweißnahtbreite erreicht werden.

Auch für die Wärmekontakt-und Wärmeimpulsverfahren sind die Bedingungen auf die Materialien einzustellen. So liegt die optimale Heizelement-temperatur für das bevorzugt eingesetzte Polysulfon zwischen 250 und 270°C, die Schweißzweiten zwischen 2 und 6 sec.

Beim Wärmeimpulsschweißverfahren sind für die eingesetzten temperaturbeständigen Thermoplaste hohe Schweißleistungen erforderlich. Sie liegen bei 50 W/cm.

Die mit dem Membranfenster 3 versehene Abdeckung 5 wird dann auf den Behälter aufgeschweißt. Dabei ist die Schweißnaht so auszuführen, daß sie dicht ist, die Abdeckung· aber komplett von Hand abgezogen werden kann. Das geschieht entweder durch eine entsprechend - schmale Schweißnaht oder eine Schweißnaht mit einer Einkerbung, entlang der die Abdeckung leicht aufzureißen ist.

Bei Verwendung von Polysulfon als Fenstermaterial kann das Verschweißen des Fenstermaterials mit der Abdeckfolie 4 und deren Verschweißung mit dem Behälter 1 in einem Arbeitsgang durchgeführt werden. Dabei weist der Fensterzuschnitt die gleiche äußere Kontur wie die Abdeckfolie auf, so daß sie unter Zwischenlage zusammen mit der Abdeckfolie mit dem Behälterflansch 7 verschweißt werden kann.

Für eine leichtere Handhabung des Behälters beim Entfernen der Abdeckung ist bei einer weiteren Ausführungsfgorm vorgesehen, das Material der Abdeckung 5 mit zwei im Abstand parallel und entlang einer Solltrennlinie verlaufenden Aufreißfäden zu versehen.

**Ansprüche**

1. Behälter zum Aufbewahren und Sterilisieren von medizinischem Material, gekennzeichnet durch eine die Öffnung (6) des Behälters (1) hermetisch abschließende und von diesem abziehbare Abdeckung (5) aus folienartigem Material, welche eine Öffnung (2) mit einer mit dem Material der Abdeckung verschweißbaren porösen Membran (3) aufweist, die undurchlässig ist für biologische Keime und durchlässig ist für sterilisierende Mittel.

2. Behälter nach Anspruch 1, dadurch gekennzeichnet, daß sowohl die Membran (3) mit dem Material der Abdeckung (5) als auch die Abdeckung mit dem Behälter (1) verschweißt sind.

3. Behälter nach Anspruch 1, dadurch gekennzeichnet, daß die Abdeckung (5) aus einer Thermoplastfolie besteht.

4. Behälter nach Anspruch 3, dadurch gekennzeichnet, daß die Thermoplastfolie faserverstärkt ist.

5. Behälter nach Anspruch 1, dadurch gekennzeichnet, daß die poröse Membran (3) aus Papier besteht.

6. Behälter nach Anspruch 1, dadurch gekennzeichnet, daß die poröse Membran (3) aus Polysulfon besteht.

7. Behälter nach Anspruch 1, dadurch gekennzeichnet, daß das Material der Abdeckung (5) mit zwei im Abstand parallel verlaufenden Aufreißfäden versehen ist.